(19) Europäisches Patentamt

European Patent Office

Office européen des brevets

(11) Veröffentlichungsnummer: **0 350 908 B1**

(12) **EUROPÄISCHE PATENTSCHRIFT**

(45) Veröffentlichungstag der Patentschrift: **07.06.95**

(21) Anmeldenummer: **89112815.9**

(22) Anmeldetag: **13.07.89**

(51) Int. Cl.⁶: **C12P  13/00**

(54) **Verfahren zur Herstellung von S-Cyanhydrinen.**

(30) Priorität: **14.07.88 DE 3823866**
          **29.05.89 DE 3917374**

(43) Veröffentlichungstag der Anmeldung:
   **17.01.90 Patentblatt  90/03**

(45) Bekanntmachung des Hinweises auf die
   Patenterteilung:
   **07.06.95 Patentblatt  95/23**

(84) Benannte Vertragsstaaten:
   **AT BE CH DE FR GB IT LI NL**

(56) Entgegenhaltungen:
   **EP-A- 0 326 063**
   **DE-B- 1 300 111**

   **CHEMICAL ABSTRACTS, Band 66, Nr. 23, 05**
   **Juni 1967, Columbus, OH (US); C.H. MAO et**
   **al., Seite 9541, Nr. 102048b&NUM;**

   **CHEMICAL ABSTRACTS, Band 76, Nr. 11, 13**
   **März 1972, Columbus, OH (US); M.K. SEELY**
   **et al., Seite 156, Nr. 55737v&NUM;**

   **ANGEWANDTE CHEMIE, Int. Ed. Engl., Band**
   **26, Nr. 5, 1987; Seiten 458-460&NUM;**

   **ANGEWANDTE CHEMIE, Band 99, Nr. 5, 1987;**

   **Seiten 491-492&NUM;**

   **PESTIC. SCI., Nr. 9, 1978; Seiten**
   **112-116&NUM;**

(73) Patentinhaber: **FORSCHUNGSZENTRUM JÜ-**
   **LICH GMBH**
   **Wilhelm-Johnen-Strasse**
   **D-52425 Jülich (DE)**

   Patentinhaber: **Degussa Aktiengesellschaft**
   **Weissfrauenstrasse 9**
   **D-60311 Frankfurt (DE)**

(72) Erfinder: **Kula, Maria-Regina, Prof. Dr.**
   **Selgenbusch 12**
   **D-5162 Hambach-Niederzier (DE)**
   Erfinder: **Niedermeyer, Uwe, Dr.**
   **Düffelstrasse 8**
   **D-5303 Walberberg (DE)**
   Erfinder: **Stürtz, Ingeborg Maria**
   **Uferstrasse 22**
   **D-5180 Eschweiler (DE)**

EP 0 350 908 B1

**Beschreibung**

Die Erfindung bezieht sich auf ein Verfahren zur Herstellung von S-Cyanhydrinen durch Umsetzung der entsprechenden Oxoverbindungen mit Blausäure.

Optisch aktive Cyanhydrine besitzen eine große Bedeutung für die Gewinnung von optisch aktiven $\alpha$-Aminoalkoholen, $\alpha$-Hydroxycarbonsäuren, Heterocyclen und Pyrethroid-Insektiziden.

Bekannt ist die Herstellung von R-Cyanhydrinen mit Hilfe chiraler Katalysatoren aus den entsprechenden Aldehyden mit Blausäure, insbesondere unter Verwendung von R-Oxynitrilase (4.1.2.10) aus Prunus amygdalus.

Diese enzymatische Reaktion mit der seit langem (seit 1908) bekannten R-Oxynitrilase (4.1.2.10) wurde in den 60-iger Jahren von Becker (JACS 88 (1966) 4299) näher untersucht, der in der DE-PS 1 300 111 die Herstellung von optisch aktiven Cyanhydrinen mit an Ionenaustauschern gebundener Oxynitrilase bei pH 5,4 beschreibt.

Nach Effenberger et al (Angew. Chem. 99 (1987) 491) soll die enzymatische Umsetzung in organischen, mit Wasser nicht mischbaren Lösungsmitteln durchgeführt werden, um die zu Racematen führende chemische Konkurrenzreaktion zu unterdrücken.

Die enzymatische Herstellung von optisch reinen S-Cyanhydrinen ist dagegen bislang nicht bekannt.

Es wurde nun gefunden, daß die aus Sorghum bicolor erhältliche Oxynitrilase (4.1.2.11) für die enzymatische Gewinnung von S-Cyanhydrinen brauchbar ist.

Das erfindungsgemäße Verfahren zur Herstellung von S-Cyanhydrinen durch Umsetzung von Oxoverbindungen mit Blausäure ist demgemäß dadurch gekennzeichnet, daß man die Umsetzung enzymatisch mit Hilfe von Oxynitrilase (4.1.2.11) aus Sorghum bicolor durchführt.

Die Oxynitrilase (4.1.2.11) ist zwar bereits seit längerer Zeit bekannt (E. Bové et al, J. Biol. Chem. 236 - (1961) 207) und ihre Reaktivität anhand der enzymatischen Spaltung von Cyanhydrinen untersucht worden (C.-H. Mao et al, Phytochemistry 6 (1967) 473). Daß mit dieser Oxynitrilase S-Cyanhydrine mit hoher optischer Reinheit erhalten werden können, war jedoch bisher unbekannt.

Besonders untersucht wurde die enzymatische Umsetzung von aromatischen Aldehyden, insbesondere von Benzaldehyd und seinen Derivaten mit Blausäure.

Ferner kann die enzymatische S-Cyanhydrin-Bildung mit Vorteil für die Erzeugung optisch aktiver Aldonsäurenitrile durch Umsetzung von Aldosen, insbesondere Aldehydzuckern mit 3 bis 9 C-Atomen mit Blausäure in Anwesenheit von S-Oxynitrilase ausgenutzt werden, wobei interessante, um 1 C-Atom vermehrte Aldonsäurenitrile entstehen, die sich leicht zur Aldonsäure verseifen oder zum Polyhydroxyamin hydrieren lassen. Besonders bevorzugt wird die Umsetzung von Aldhosen der allgemeinen Formel R-CHO, in der R = -[CHOH]$_n$-CH$_2$OH mit n = 1 bis 7 ist.

Für die enzymatische Gewinnung von S-Cyanhydrinen mit besonders hoher optischer Reinheit ist es notwendig, die rein chemische Reaktion der Aldehyde mit Blausäure, die zu Racematen führt und daher die optische Reinheit der Produkte beeinträchtigen würde, zu unterdrücken. Außerdem neigen chirale Cyanhydrine zur Racemisierung, wodurch die optische Reinheit des Produktes weiter vermindert wird.

Vorzugsweise wird daher bei pH-Werten und Temperaturen gearbeitet, bei denen die Konkurrenzreaktionen und Racemisierungen unterdruckt werden können. Dabei bestimmt die Reaktivität des Aldehyds bzw. des Cyanhydrins, wie tief der pH-Wert abzusenken ist, damit diese Begleitreaktionen zu vernachlässigen sind.

Es wurde nämlich festgestellt, daß es möglich ist, mit der Oxynitrilase (4.1.2.11) aus Sorghum bicolor in einem solchen aciden pH-Bereich zu arbeiten, daß die enzymatische Reaktion bei weitem überwiegt.

Das Enzym besitzt nämlich bei pH 4,9 das Reaktionsoptimum und hat bei pH 3,25 noch 40 % der maximalen Aktivität. Durch diese pH-Toleranz der Oxynitrilase (4.1.2.11) wird es also möglich, zu Produkten mit hoher optischer Reinheit zu gelangen

Dabei bieten die Variation der Substratkonzentrationen und/oder der Reaktionstemperatur zusätzliche Möglichkeiten, die chemische Begleitreaktion relativ zur enzymatischen Umsetzung zu hemmen, so daß ein gewisser Spielraum für mögliche günstigste Bedingungen gegeben ist.

Vorzugsweise wird mit pH-Werten unter 6,0, insbesondere unter 4,5 gearbeitet.

Die Reaktionstemperatur liegt zweckmäßigerweise im Bereich von -5° bis +50°C.

Die Reaktion erfolgt insbesondere in wäßrigem Milieu, das maximal 40 %, insbesondere nicht mehr als 25 % organisches Cosolvens enthalten soll.

Zu beachten ist dabei, daß die Oxynitrilasen nur dann genügend Aktivität besitzen, wenn mit der pH-Wert-Senkung auch eine Reduktion des Cosolventiengehalts einhergeht. Da die Enzymaktivität jedoch durch die Anwesenheit von auch nur geringen Mengen an organischen Lösungsmitteln (z. B. Ethanol) erheblich vermindert wird, sollte vorzugsweise ohne jeglichen Lösungsmittelzusatz gearbeitet werden.

Die Umsetzungen können mit dem nativen Enzym, aber auch mit dem immobilisierten Enzym durchgeführt werden. In beiden Formen ist das Enzym zeitlich stabil, so daß es auch in kontinuierlicher Arbeitsweise genutzt werden kann.

Eine kontinuierliche Reaktion ist zweckmäßigerweise durchführbar. Insbesondere wird im Enzymmembranreaktor gearbeitet. Der Reaktionsverlauf kann in Anbetracht der unterschiedlichen Drehwerte von Edukt (z.B. Monosaccharid) und Produkt (z.B. Aldonsäurenitril) besonders elegant mit einem Polarimeter verfolgt werden.

Die Trennung von Edukt und Produkt gelingt mit Hilfe der Dünnschichtchromatographie unter Verwendung polarer Laufmittel, wie z.B. Butanol : Pyridin : Wasser im Verhältnis 6 : 4 : 3 (v : v : v). Eine quantitative Trennung und Bestimmung der Diastereomere mit Hilfe von HPLC kann ebenfalls herangezogen werden.

Nachfolgend wird die Erfindung anhand von Beispielen beschrieben.

Beispiel 1

52 mg (0,5 mmol) para-Hydroxy-benzaldehyd wurden in 9,4 ml 50 mM Citratpuffer von pH 3,75 gelöst und auf 20°C temperiert. Hierzu wurden 500 µl (S)-Oxynitrilase-Lösung und 800 µl 4,2 M wäßrige HCN-Lösung hinzugegeben. Die eingesetzte (S)-Oxynitrilase-Lösung hatte eine Aktivität von 84 U/ml, wobei 1 U die Bildung von einem µmol/min para-Hydroxymandelsäurenitril bei 20°C und pH 3,75 katalysiert. Die Reaktion wurde polarimetrisch (λ = 578 nm) verfolgt. Nach 15 bis 30 Min. stellte sich ein konstanter Drehwert ein, und die Reaktion war beendet. Danach wurde das Reaktionsgemisch viermal mit 10 ml Diethylether extrahiert. Die vereinigten organischen Phasen wurden über Natriumsulfat getrocknet und das Lösungsmittel am Rotationsverdampfer abgezogen. Anschließend wurde der Rückstand dreimal mit je 10 ml Pentan gewaschen und das Produkt im Vakuum getrocknet.

| Chem. Ausbeute: | 64,8 mg (87 % d. Th.) |
| Optische Reinheit: | 99 % ee |

Die Bestimmung der optischen Reinheit erfolgte als N,O-Bis-(Pentafluorpropionyl)-2-amino-1-(p-hydroxyphenyl)ethanol-Derivat des Mandelsäurenitrils kapillargaschromatographisch nach H. Frank et al. (J. Chromatogr. 146 (1987) 197-206) auf einer chiralen Trennphase (FS-Chirasil-Val, 25 m * 0,32 mm).

Die Derivatisierung wurde wie folgt vorgenommen: 1 - 2 mg des Mandelsäurenitrils wurden mit 250 µl einer 1 M Diboranlösung (in Tetrahydrofuran) in Chloroform bei Zimmertemperatur in 30 Min reduziert. Nach Hydrolyse des überschüssigen Diborans mit einigen Tropfen Ethanol und Abziehen des Lösungsmittels wurde der erhaltene Aminoalkohol direkt mit 20 µl Pentafluorpropionsäureanhydrid in Methylenchlorid bei Zimmertemperatur in 15 Min acyliert. Schließlich wurde überschüssiges Anhydrid am Rotationsverdampfer abgezogen, der Rückstand mit Methylenchlorid wieder aufgenommen und gaschromatographisch analysiert.

Beispiel 2

Entsprechend Beispiel 1 wurden 61 mg (0,5 mmol) meta-Hydroxy-benzaldehyd bei pH 3,25 mit 450 µl 4,2 M wäßriger HCN-Lösung umgesetzt. Nach 30 bis 40 Minuten war die Reaktion beendet. Die Isolierung des (S)-3-Hydroxymandelsäurenitrils erfolgte wie in Beispiel 1 beschrieben.

| Chem. Ausbeute: | 67 mg (90 % d. Th.) |
| Optische Reinheit: | 98 % ee |

Die Bestimmung der optischen Reinheit erfolgte über das N,O-Bis-Pentafluorpropionyl-2-amino-1-(m-hydroxyphenyl)ethanol gemäß Beispiel 1.

Beispiel 3

Entsprechend Beispiel 1 wurden 60 mg (0,5 mmol) meta-Methyl-benzaldehyd mit 475 µl 4,2 M wäßriger HCN-Lösung und 500 µl Oxynitrilase-Lösung bei pH 3,25 umgesetzt. Nach ca. 45 Min war die Reaktion beendet. Die Aufarbeitung erfolgte analog Beispiel 1 unter Gewinnung von (S)-3-Methylmandelsäurenitril.

| Chem. Ausbeute: | 59 mg (80 % d. Th.) |
|---|---|
| Optische Reinheit: | 96 % ee |

Die Bestimmung der optischen Reinheit erfolgte über das N,O-Bis-Pentafluorpropionylesteramid des entsprechenden Aminoalkohols gemäß Beispiel 1.

Beispiel 4

Entsprechend Beispiel 1 wurden 53 mg (0,5 mmol) Benzaldehyd mit 475 $\mu$l 4,2 M wäßriger HCN-Lösung und 1500 $\mu$l (S)-Oxynitrilaselösung bei pH 3,25 umgesetzt. Nach ca. 45 Min war die Reaktion beendet. Die Aufarbeitung erfolgte analog zu Beispiel 1.

| Chem. Ausbeute: | 48 mg (80 % d. Th.) |
|---|---|
| Optische Reinheit: | 96 % ee |

Die Bestimmung der optischen Reinheit erfolgte über das N,O-Bis-Pentafluorpropionylesteramid gemäß Beispiel 1.

Die eingesetzte S-Oxynitrilase-Lösung hatte eine Aktivität von 84 U/ml, wobei 1 U die Bildung von einem $\mu$M/min p-Hydroxymandelsäurenitril bei 20 °C und pH 3,75 katalysiert.

Beispiel 5

Kontinuierliche Produktion von (S)-para-Hydroxymandelsäurenitril im Rührkesselreaktor mit immobilisierter (S)-Oxynitrilase

Eine Übertragung des Reaktionsprinzips auf die Bildung größerer Produktmengen in kontinuierlicher Arbeitsweise erfolgte mit an Eupergit® C (Röhm, Darmstadt) immobilisierter (S)-Oxynitrilase.

In kontinuierlicher Arbeitsweise wurde in einem 10 ml Reaktor über 72 h mit einer Raumzeitausbeute von 57,9 g/(l d) (S)-para-Hydroxymandelsäurenitril produziert.

Charakteristische Reaktionsdaten:

| para-Hydoxybenzaldehyd: | 21 mM | |
|---|---|---|
| HCN: | 400 mM | |
| Na-Citrat: | 45 mM | pH 3,75 |
| Verweilzeit: | 3960 sec | |
| Katalysatorkonzentration: | 0,15 g/ml | |
| Laufzeit: | 72 h | |
| Enzymdesaktivierung: | ca. 3 %/d | |
| Reaktionsumsatz: | 85 % | |
| Enantiomerenüberschuß: | > 98 % | |
| Temperatur | 20 °C | |

Beispiel 6

Herstellung von Aldonsäurenitril

Ansatz:
150 mg D(-)-Arabinose (100 mmol); Drehwert:
$[\alpha]_D^{20}$ = -103,01 [˙. ml/g • dm] (c = 3,75/Citratpuffer)
4 ml Natriumcitratpuffer (50 mM; pH 3,75)
100 U Enzymstammlösung ((S)-Oxynitrilase)
50 $\mu$l Blausäure
Die Arabinose wurde in 4 ml Natriumcitratpuffer gelöst und zu dieser Lösung die Enzymlösung sowie die

EP 0 350 908 B1

Blausäure hinzugegeben und die Reaktionsmischung 5 Tage lang bei Raumtemperatur geschüttelt.
Die Reaktion wurde mit Hilfe des Drehwertes und der Dünnschichtchromatographie verfolgt.
Die Rf-Werte des an Kieselgelplatten mit Hilfe des oben genannten Laufmittels voneinander getrennten Edukts und Produkts lagen bei 0,68 bzw. 0,81.
Drehwert der Produktlösung:
$[\alpha]_D^{20}$ = -45,54 [˙. ml/g • dm] (c = 3,75/Citratpuffer)

**Patentansprüche**

1. Verfahren zur Herstellung von S-Cyanhydrinen durch Umsetzung der entsprechenden Oxoverbindungen mit Blausäure,
**dadurch gekennzeichnet,**
daß man die Umsetzung enzymatisch mit Hilfe von Oxynitrilase (4.1.2.11) aus Sorghum bicolor durchführt.

2. Verfahren nach Anspruch 1,
**dadurch gekennzeichnet,**
daß man in wäßrigem Milieu bei pH-Werten unter 6,0, insbesondere unter 4,5 arbeitet.

3. Verfahren nach Anspruch 1 oder 2,
**dadurch gekennzeichnet,**
daß man Aldehyde, insbesondere aromatische Aldehyde umsetzt.

4. Verfahren nach Anspruch 3,
**dadurch gekennzeichnet,**
daß man Benzaldehyd oder seine Derivate umsetzt.

5. Verfahren nach Anspruch 1 oder 2,
**dadurch gekennzeichnet,**
daß man als Oxoverbindungen Aldosen verwendet und daraus die um 1 C-Atom vermehrten Aldonsäurenitrile herstellt.

6. Verfahren nach Anspruch 5,
**dadurch gekennzeichnet,**
daß man eine Aldose der allgemeinen Formel R-CHO umsetzt, bei der R ein Polyolrest der Formel -[CHOH]$_n$-CH$_2$OH mit n = 1 bis 7 ist.

**Claims**

1. Process for producing S-cyanohydrines by conversion of respective oxo-compounds with hydrocyanic acid, **characterized in that** the conversion is carried out enzymatically with the aid of oxynitrilase (4.1.2.11) of sorghum bicolour.

2. Process according to claim 1, **characterized in that** work is carried out in an aqueous milieu at pH values below 6.0, in particular below 4.5.

3. Process according to claim 1 or 2, **characterized in that** aldehydes, in particular aromatic aldehydes, are converted.

4. Process according to claim 3, **characterized in that** benzaldehyde or its derivatives are converted.

5. Process according to claim 1 or 2, **characterized** in that aldoses are used as oxo-compounds and therefrom are produced aldonic acid nitriles increased by 1 C-atom.

6. Process according to claim 5, **characterized in that** that an aldose of the general formula R-CHO is converted, where R is a polyol residue of the formula

-[CHOH]$_n$-CH$_2$OH with n = 1 to 7.

5

**Revendications**

1. Procédé de préparation de S-cyanhydrines par réaction des composés oxo correspondants avec l'acide cyanhydrique, caractérisé en ce qu'on effectue la réaction par voie enzymatique à l'aide d'oxynitrilase (4.1.2.11) provenant de *Sorghum bicolor*.

2. Procédé selon la revendication 1, caractérisé en ce qu'on travaille en milieu aqueux à des valeurs de pH inférieures a 6,0, en particulier inférieures à 4,5.

3. Procédé selon la revendication 1 ou 2, caractérisé en ce qu'on fait réagir des aldéhydes, en particulier des aldéhydes aromatiques.

4. Procédé selon la revendication 3, caractérisé en ce qu'on fait réagir le benzaldéhyde ou ses dérivés.

5. Procédé selon la revendication 1 ou 2, caractérisé en ce qu'on utilise des aldoses comme composés oxo, et, à partir de ceux-ci, on prépare les nitriles aldoniques augmentés d'un atome de carbone.

6. Procédé selon la revendication 5, caractérisé en ce qu'on fait réagir un aldose répondant à la formule générale R-CHO, dans laquelle R est un reste polyol répondant à la formule $-[CHOH]_nCH_2OH$ avec n = 1 à 7.